# EUROPEAN PATENT SPECIFICATION

(11) **EP 1 392 627 B1**
(45) Date of publication and mention of the grant of the patent: **05.08.2009**
(21) Application number: 02748718.0
(22) Date of filing: 23.05.2002
(51) Int. Cl.: C07C 2/58

(54) **PROCESS FOR THE CATALYTIC ALKYLATION OF HYDROCARBONS**
VERFAHREN ZUR KATALYTISCHEN ALKYLIERUNG VON KOHLENWASSERSTOFFEN
PROCESSUS D'ALKYLATION CATALYTIQUE D'HYDROCARBURES

(30) Priority: 08.06.2001 EP 01202204
(43) Date of publication of application: 03.03.2004
(73) Proprietor: Albemarle Netherlands B.V., 3818 LH Amersfoort (NL)
(72) Inventor: VAN BROEKHOVEN, Emanuel, Hermanus, NL-1141 DN Monnickendam (NL); SONNEMANS, Johannes, Wilhelmus, Maria, NL-3761 DD Soest (NL); ZUIJDENDORP, Stephan, NL-1501 VW Zaandam (NL)
(74) Representative: Rasser, Jacobus Cornelis
(86) International application number: PCT/EP2002/005758
(87) International publication number: WO 2002/100807

(56) References cited:
- EP-A- 1 070 694
- WO-A-98/23560
- US-A- 5 523 503

## Description

This invention relates to a process for the alkylation of hydrocarbons over a solid acid catalyst, and more in particular to a process in which the catalyst is also regenerated.

Within the framework of the present invention, the term alkylation refers to the reaction of an alkylatable compound, such as an aromatic or a saturated hydrocarbon, with an alkylation agent, such as an olefin. Without limiting its scope, we will further illustrate the invention by discussing the alkylation of saturated hydrocarbons, in general branched saturated hydrocarbons, with an olefin to give highly branched saturated hydrocarbons with a higher molecular weight.

WO 98/23560 discloses such a process for alkylating hydrocarbons. This process uses a solid acid catalyst, which catalyst is regenerated with hydrogen under mild conditions before there is any substantial decrease of its activity.
During this regeneration hydrocarbon, preferably the alkylatable compound, is present.

This process uses one reactor which cycles between the alkylation mode and the regeneration mode. So, during regeneration the alkylate production is on hold. Moreover, in this process preference is given to the introduction of washing steps between the reaction step and the regeneration step as well as between the regeneration step and the reaction step, in which washing steps the catalyst is washed with a saturated hydrocarbon, preferably the alkylatable compound.

An alkylation process which includes mild regeneration of the catalyst is also described in US 5,523,503, which discloses the alkylation of hydrocarbons, i.e. isobutane, in an apparatus comprising at least three reactors. The reactors cycle between a zone in which alkylation is performed and a zone where the catalyst is regenerated. Cycling of the reactors between the two zones occurs by periodically advancing the locations at which the first feed stream and the regenerant stream enter the process in a manner such as to simulate the cocurrent movement of the beds of catalysts relative to the direction of the liquid phase reactant flow.
In this process, the points of feed and regenerant injection move along the reactors. Such a process requires a lot of valves and tubing. A second disadvantage of this process is the need for two separate isobutane-containing streams: a feed stream and a regenerant stream. A third disadvantage is that before cycling a reactor from the regeneration to the alkylation zone and *vice versa,* the reactor needs to be flushed in order to remove alkylate.

It would be desirable to be able to operate such a combined process of alkylation and mild regeneration continuously, meaning that the alkylate production and the catalyst regeneration can be conducted at the same time, with washing steps not being required. Moreover, it would be desirable for such a process to only require a minimum of tubing and valves. The process according to the invention provides the means for such a continuous process.

The invention relates to a process comprising (i) reacting an alkylatable compound with an alkylation agent over a solid acid alkylation catalyst to form an alkylate, and (ii) regenerating said catalyst under mild regeneration conditions in the presence of hydrogen and hydrocarbon. The process is
characterised in that the hydrocarbon present during regeneration comprises at least a portion of the formed alkylate.

It has surprisingly been found that the alkylate remains essentially unconverted during regeneration of the catalyst with hydrogen. This is indeed surprising, because it is common knowledge that saturated hydrocarbons are easily converted in the presence of hydrogen and solid acid catalysts with a hydrogenating function. Examples of such conversions are hydrocracking, isomerisation, etc.
The fact that in the process according to the invention the alkylate remains essentially unconverted in the presence of hydrogen and the solid acid alkylation catalyst means that there is no need to remove the alkylate from the reactor system before regeneration can be started.
Moreover, the entire alkylate-containing product stream can be present during regeneration. Therefore, this process requires only one stream of alkylatable compound, whereas the process according to US 5,523,503 needs two such streams: one for the reactors in the alkylation mode and the other for the reactors in the mild regeneration mode.

The fact that alkylate can be present during mild regeneration makes it possible to design a relatively simple and economically favourable continuous process for alkylation combined with mild regeneration of the catalyst. Such a process requires an apparatus comprising two or more solid acid alkylation catalyst-containing reactors. At least one of these two or more reactors operates in the alkylation mode - comprising reacting the alkylatable compound and the alkylation agent to form an alkylate-containing product stream - and at least one of these two or more reactors operates in the mild regeneration mode - comprising contacting said solid acid alkylation catalyst with hydrogen in the presence of formed alkylate.
It is to be understood that during operation in the regeneration mode there may be time periods when the catalyst is not contacted with hydrogen. Analogously, during operation in the alkylation mode there may be time periods when no alkylation agent is introduced.

In a first embodiment, alkylate is introduced by feeding at least a portion of the alkylate-containing product stream to the reactor. The reactors periodically cycle back and forth between the alkylation mode and the mild regeneration mode. Finally, the alkylate is recovered from the effluent of the reactor operating in the mild regeneration mode or, if there are more reactors operating in the mild regeneration mode, at least one of the reactors operating in this mode. If only a portion of the alkylate-containing product stream is contacted with the solid acid alkylation catalyst during mild regeneration, alkylate is also recovered from the remaining portion of the alkylate-containing product stream, i.e. the portion which bypasses the reactor(s) operating in the mild regeneration mode.

In a second embodiment, the reactor(s) in the mild regeneration mode is (are) bypassed by the alkylate-containing product stream, and regeneration is performed with hydrogen in the presence of alkylate still present in the reactor after cycling from the alkylation mode to the regeneration mode. The reactors periodically cycle back and forth between the alkylation mode and the mild regeneration mode. Finally, the alkylate is recovered from the alkylate-containing product stream.

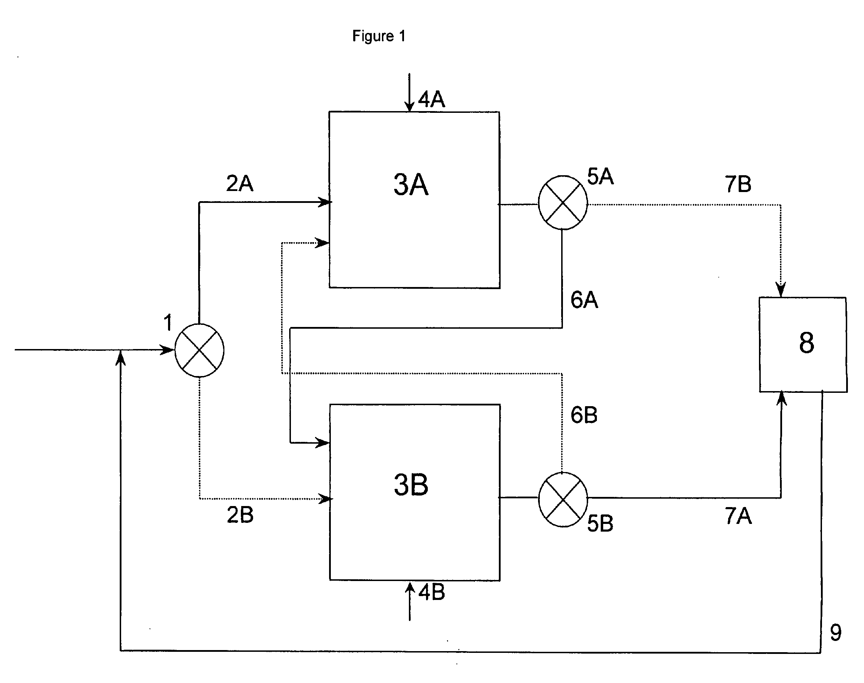

Figure 1 shows a schematic lay-out of an apparatus suitable for carrying out the process according to the invention. By way of example, this figure displays only two solid acid alkylation catalyst-containing reactors: one in the alkylation mode and the other in the regeneration mode. Note that according to the invention, the apparatus can contain more than one reactor in the alkylation mode and more than one reactor in the regeneration mode. Preferably, the apparatus contains the same number of reactors in the alkylation mode as it does in the regeneration mode. Preferably, the apparatus contains 1-3 reactors in each mode.

The reactors contain the solid acid alkylation catalyst and are equipped with standard equipment known in the art for measuring temperature, pressure, and flow. The catalyst can be present in any type of catalyst bed, for instance a fluidised bed, a fixed bed or suspended in a slurry. Moreover, the reactors can contain more than one catalyst bed. This allows for the use in one reactor of catalyst beds with different catalyst compositions and/or of different size. For instance, the most downstream catalyst bed can be larger, i.e. contain a larger amount of catalyst, and/or can contain a more active catalyst than the upstream catalyst beds in the same reactor. This can be useful for reaching complete conversion of the alkylation agent, thereby preventing breakthrough of this compound.
When using at least two catalyst beds in a reactor, it is preferred that each of these catalyst beds is provided with its own addition of alkylation agent, so that the total amount of olefin needed in the reactor is spread over several injection points. This results in higher molar ratios of alkylatable compound to alkylation agent in the reactor. The injection points are located below each of the catalyst beds, i.e. below the most upstream bed and below each of the subsequent catalyst beds. Before entering the catalyst bed, the injected alkylation agent is preferably mixed with the alkylatable compound already present in the reactor.

Alternatively, it is possible to use at least two catalyst beds in a reactor and inject alkylation agent below each of the catalyst beds except for the most downstream one. This can also stimulate complete conversion of the alkylation agent in the reactor and prevent breakthrough of this compound. Furthermore, it is possible to use at least two catalyst beds in a reactor and inject different alkylation agents below the different catalyst beds. For instance, butene can be added to the first two beds, whereas propene is added to the third catalyst bed.

The apparatus of Figure 1 comprises a reactor 3A, which is in the alkylation mode. To this reactor, alkylatable compound is fed via valve system 1 and through duct 2A, and the alkylation agent is fed through inlet 4A. The two components can (partially) react to form an alkylate.

Valve systems 1, 5A, and 5B of this apparatus are only schematically represented in Figure 1. Preferably, these valve systems are sets of valves and/or flow controllers which regulate the streams in ducts 2A and 2B, ducts 7B and 6A, and ducts 7A and 6B, respectively. The advantage of such sets of valves and/or flow controllers is that for instance the stream of alkylatable compound through duct 2A is not necessarily blocked the moment it is introduced into duct 2B, as will be the case when using, e.g., three-way valves. Moreover, flow controllers offer the possibility of regulating the streams flowing through the ducts.

In the process according to the first embodiment, at least a portion of the alkylate-containing product stream is led via valve system 5A and through duct 6A to reactor 3B, which is in the regeneration mode. If only a portion of the alkylate-containing product stream is led to reactor 3B, the remaining portion of the alkylate-containing product stream can be led through duct 7B to separation unit 8. If desired, alkylatable compound may be added to the portion of the alkylate-containing product stream which is led to reactor 3B. Preferably, the entire alkylate-containing product stream is led to reactor 3B.
Hydrogen is fed to reactor 3B through inlet 4B. The effluent of this reactor is subsequently led via valve system 5B and through duct 7A to separation unit 8.
The separation unit may comprise one or more separation devices. In this separation unit alkylatable compound is separated from the effluent of reactor 3B and, if only a portion of the alkylate-containing product stream is led to reactor 3B, from the remaining portion of the alkylate-containing product stream. A stream comprising at least a portion of this alkylatable compound is recycled through duct 9 to valve system 1. Note that this stream and, therefore, the alkylatable compound introduced into reactor 3A, may contain other compounds such as alkylate. Other streams leaving the separation unit comprise the alkylate and usually also comprise a stream containing "lights", such as hydrogen and/or propane, and an n-alkanes-containing stream.
The reactors 3A and 3B can cycle between the alkylation mode and the regeneration mode by, e.g., (i) switching the streams coming through inlets 4A and 4B and (ii) changing the streams flowing through the different ducts. In this mode, hydrogen is fed to reactor 3A and the alkylation agent is fed to reactor 3B. Alkylatable compound is fed through duct 2B to reactor 3B, at least a portion of the alkylate-containing product stream is led via valve system 5B and through duct 6B to reactor 3A. If only a portion of the alkylate-containing product stream is led to reactor 3A, the remaining portion of the alkylate-containing product stream is led via duct 7A to separation unit 8, and the stream coming out of reactor 3A is led via valve system 5A and through duct 7B to separation unit 8.

In the process according to the second embodiment, the alkylate-containing product stream is led via valve system 5A and through duct 7B to separation unit 8. In this separation unit alkylatable compound is separated from the alkylate-containing product stream. A stream comprising at least a portion of this alkylatable compound can be recycled through duct 9.
No alkylate-containing product stream is fed to reactor 3B. Alkylate is still present in this reactor from when it operated in the alkylation mode. Hydrogen is introduced into reactor 3B through inlet 4B. In principle, reactor 3B will be isolated from the system, meaning that no streams leave the reactor via valve system 5B during the regeneration period. However, it may be necessary to relieve the pressure from the reactor via, e.g., duct 7A.
The reactors 3A and 3B can cycle between the alkylation mode and the regeneration mode by, e.g., (i) switching the streams coming through inlets 4A and 4B and (ii) changing the streams flowing through the different ducts. In this mode, hydrogen is fed to reactor 3A and the alkylation agent is fed to reactor 3B. Alkylatable compound is fed through duct 2B to reactor 3B, the alkylate-containing product stream is led via duct 7A to the separation unit 8.

The skilled person will appreciate that both embodiments allow for continuous operation.

The process is typically performed under conditions such that at least a portion of the alkylation agent and the alkylatable compound are in the liquid phase or the supercritical phase. In general, the process according to the invention is performed at a temperature in the range of 233 to 523 K, preferably in the range of 293 to 423 K, more preferably in the range of 338 to 368 K, and a pressure in the range 1 to 100 bar, preferably 5 to 40 bar, more preferably 15 to 30 bar.

In general, the mild regeneration step is carried out at a temperature in the range of 233 to 523 K and a pressure from 1 to 100 bar. Although the regeneration and alkylation steps can be performed at different temperatures and pressures, it is preferred for the regeneration temperature, expressed in K, and the regeneration pressure, not to differ from the reaction temperature and the reaction pressure by more than 20%, more preferably not by more than 10%, still more preferably not by more than 5%. Most preferably, the regeneration temperature and pressure and the reaction temperature and pressure are essentially the same.

The molar ratio of alkylatable compound to alkylation agent in the total feed in the reactor preferably is higher than 5:1, more preferably higher than 50:1. Higher molar ratios are considered preferred for performance reasons, because they generally yield an increase in octane number and stability. The upper limit for this ratio is determined by the type of process applied, and by the process economics. It is not critical, and may be as high as 5,000:1. Generally, figures of, e.g., 1,000:1 or lower are preferred. These high molar ratios can be obtained in various ways known to the skilled person, e.g., by multiple inlets for alkylation agent or by internal recycling of reactor contents. At this moment a molar ratio of alkylatable compound to alkylation agent of 150-750:1 is considered most preferred.
The feed rate (WHSV) of the alkylation agent generally is in the range of 0.01 to 5, preferably in the range of 0.05 to 0.5, more preferably in the range of 0.1 to 0.3 gram of alkylation agent per gram of catalyst per hour.

The catalyst is regenerated by being contacted with hydrogen in the presence of formed alkylate. Typically, the hydrogen will be dissolved in the alkylate-containing mixture, i.e. the alkylate-containing product stream (embodiment 1) or the mixture containing the formed alkylate which is still present after cycling the reactor(s) from the alkylation mode to the regeneration mode (embodiment 2). Preferably, the solution contains at least 10% of the saturation concentration of hydrogen, said saturation concentration being defined as the maximum quantity of hydrogen which can be dissolved in the alkylate-containing mixture at regeneration temperature and pressure. Depending on the applied feed rates it may be more preferred for the solution to contain at least 50% of the saturation concentration, even more preferably at least 85%. At relatively low feed rates it is generally preferred to have as saturated a solution of hydrogen in the mixture as possible.

The frequency of cycling the reactors between the alkylation mode and the regeneration mode, i.e. the regeneration frequency, depends on a number of conditions, including the nature of the catalyst, the reaction and regeneration conditions, and the amount of hydrogen present during the regeneration step. Preferably, the regeneration is performed before there is any substantial decrease of catalytic activity. Such a decrease can be observed by breakthrough of alkylation agent, which can be measured by analysing the concentration of alkylation agent in the alkylate-containing product stream. By cycling the reactors between the alkylation and regeneration modes prior to breakthrough of alkylation agent, it is possible to obtain a product of nearly constant composition in a high yield. Typically, the reactors are switched between the alkylation and the regeneration mode with a frequency in the range of once per 10 hours to 10 times per hour, preferably once per 3 hours to 3 times per hour and even more preferably once per 2 hours to 2 times per hour.

Optionally, the reactors may be flushed with a hydrocarbon-containing stream, e.g., an alkylatable compound-containing stream or an alkylate-containing product stream, before cycling of the zones from the alkylation to the regeneration mode or *vice versa,* in order to prevent hydrogen from being contacted with olefin, which would result in the formation of unwanted alkanes.

The apparatus may comprise one or more additional solid acid alkylation catalyst-containing reactors which can replace one or more of the two or more reactors in the alkylation and regeneration modes. Preferably, the apparatus contains one such additional reactor. The availability of such additional reactors makes the process very flexible. If, owing to circumstances, the catalyst in one or more of the two or more reactors deactivates to an unacceptable extent during the process, it can be replaced with one or more additional catalyst-containing reactors. The deactivated catalyst can then be regenerated by contacting it with hydrogen at high temperature in the gas phase to recover its original activity without affecting the ongoing alkylation process. After this high-temperature regeneration the reactor containing the high-temperature regenerated catalyst can serve as said additional reactor.

The high-temperature regeneration is effected at a temperature of at least 423 K, preferably in the range of 423-873 , more preferably 473-673 K. To effect a long-term process on a commercial scale one can, e.g., carry out such a high-temperature regeneration after every 50, preferably after every 100 regenerations under mild conditions. Pilot plant experiments have shown that it is possible to effect a long-term process when the catalyst is subjected to a high-temperature regeneration after every 200-400 regenerations under mild conditions. Depending on the exact process variables on a commercial scale, this value may be higher or lower in actual practice.

Preferred alkylatable compounds to be used in the process according to the invention are isoalkanes having 4-10 carbon atoms, such as isobutane, isopentane, isohexane or mixtures thereof.
Preferred alkylation agents are olefins having 2-10 carbon atoms, preferably 2-6 carbon atoms, more preferably 3-5 carbon atoms, e.g., propene, butene, pentene. The alkylation of isobutane with butene or a mixture of butenes constitutes an attractive embodiment of the process according to the invention.

The solid acid alkylation catalyst used in the process according to the invention comprises a hydrogenating metal component and a solid acid constituent.
Examples of suitable hydrogenating metal components are constituents of the transition metals, such as metals of Group VIII of the Periodic Table, or mixtures thereof. Among these, noble metals of Group VIII of the Periodic Table are preferred. Platinum, palladium, and mixtures thereof are especially preferred. The amount of hydrogenating metal component will be dependent on its nature. When the hydrogenating metal component is a noble metal of Group VIII of the Periodic Table, the catalyst generally will contain in the range of 0.01 to 2 wt.% of the metal, preferably 0.1-1 wt.%, calculated as metal.
Examples of solid acid constituents are zeolites such as zeolite beta, MCM-22, MCM-36, mordenite, X-zeolites and Y-zeolites, including H-Y-zeolites and USY-zeolites, non-zeolitic solid acids such as silica-alumina, sulphated oxides such as sulphated oxides of zirconium, titanium, or tin, sulphated mixed oxides of zirconium, molybdenum, tungsten, etc., and chlorinated aluminium oxides. The presently preferred solid acid constituents are zeolites, including mordenite, zeolite beta, X-zeolites and Y-zeolites, including H-Y-zeolites and USY-zeolites, sulphated oxides, and chlorinated aluminium oxides. Mixtures of solid acid constituents can also be employed.

The catalyst to be used in the process according to the invention preferably comprises a hydrogenating metal component on a carrier which comprises 2-98 wt.% of solid acid constituent and 98-2 wt.% of a matrix material, calculated on the carrier. Preferably, the carrier comprises 10-90 wt.% of matrix material, and 90-10 wt.% of solid acid constituent. More preferably, the carrier comprises 10-80 wt.% of matrix material and the balance is solid acid constituent. Especially preferred is the catalyst wherein the carrier comprises 10-40 wt.% of matrix material and the balance is solid acid constituent.
In the present specification the term matrix material encompasses all components which are present in the catalyst except for the solid acid constituent and the hydrogenating metal component. Examples of suitable matrix materials are alumina, silica, days, and mixtures thereof. Matrix materials comprising alumina are generally preferred. A matrix material which consists essentially of alumina is considered most preferred at this point in time.

Preferably, the catalyst to be used in the process according to the invention has a particle size of at least 0.5 mm. Preferably, the particle size is at least 0.8 mm, more preferably at least 1.0 mm. The upper limit of the particle size preferably lies at 10 mm, more preferably at 5 mm, even more preferably at 3 mm.
In the present specification, the term particle size is defined as the average diameter of the solid part of the catalyst, as will be clear to the skilled person.

The catalyst can be prepared by processes common to the industry. These will comprise, say, shaping the solid acid constituent after mixing it with a matrix material, to form particles, followed by calcination of the particles. The hydrogenating function may, e.g., be incorporated into the catalyst composition by impregnating the carrier particles with a solution of a hydrogenation metal component.

Preferred alkylates to be produced by the process according to the invention are C₅+ alkylates with a minimum of C₉+ alkylates. The C₅+ alkylate obtained using the process according to the invention preferably has a C₉+ content of less than 30 wt.%, more preferably of less than 20 wt.%, most preferably of less than 10 wt.%. Frequent mild regeneration enables C₉+ production to be controlled at a comparatively low level.
Also, depending on the regeneration frequency, in the process according to the invention a high C₅+ alkylate yield is obtained. The process according to the invention makes it possible to obtain a C₅+ alkylate yield in excess of 200%, calculated on the weight of the consumed olefin, preferably of 204% or higher.
The quality of the alkylate product obtained in the process according to the invention can be measured by the RON of the product. The RON is a measure of the anti-knock rating of gasoline and/or gasoline constituents. The higher the RON, the more favourable the anti-knock rating of the gasoline will be. Depending on the type of gasoline engine, generally speaking a higher anti-knock rating is of advantage when it comes to the working of the engine. The product obtained in the process according to the invention preferably has a RON of 90 or higher, more preferably of 92 or higher, most preferably 94 or higher. The RON is obtained by determining, e.g., via gas chromatography, the percentage by volume of the various hydrocarbons in the product. The percentages by volume are then multiplied by the RON contribution and added up. Examples of compounds with a RON of 90 or higher are isopentane, 2,2-dimethyl butane, 2,3-dimethyl butane, trimethyl butane, 2,3-dimethyl pentane, 2,2,4-trimethyl pentane, 2,2,3-trimethyl pentane, 2,3,4-trimethyl pentane, 2,3,3-trimethyl pentane, and 2,2,5-trimethyl hexane.

## Claims

1. A process comprising (i) reacting an alkylatable compound with an alkylation agent over a solid acid alkylation catalyst to form an alkylate, and (ii) regenerating said catalyst under mild regeneration conditions in the presence of hydrogen and hydrocarbon, wherein the hydrocarbon comprises at least a portion of the formed alkylate.

2. A process according to claim 1 wherein the process is carried out in an apparatus comprising two or more solid acid alkylation catalyst-containing reactors, and in which process
(a) at least one of said two or more reactors operates in the alkylation mode, comprising reacting the alkylatable compound and the alkylation agent to form an alkylate-containing product stream,
(b) at least one of said two or more reactors operates in the mild regeneration mode, comprising contacting said solid acid alkylation catalyst with at least a portion of the alkylate-containing product stream in the presence of hydrogen,
(c) said reactors periodically cycle back and forth between the alkylation mode and the mild regeneration mode,
(d) alkylate is recovered (d1) if the entire alkylate-containing product stream is contacted with the solid acid alkylation catalyst during mild regeneration, and
(d1a) if there is only one reactor operating in the mild regeneration mode, from the effluent of this reactor,
(d1b) if there are two or more reactors operating in the mild regeneration mode, from the effluent of at least one of these reactors,
(d2) if only a portion of the alkylate-containing product stream is contacted with the solid acid alkylation catalyst during mild regeneration, from the remaining portion of the alkylate-containing product stream and
(d2a) if there is only one reactor operating in the mild regeneration mode, from the effluent of this reactor,
(d2b) if there are two or more reactors operating in the mild regeneration mode, from the effluent of at least one of these reactors.

3. A process according to claim 2 wherein in the mild regeneration mode the solid acid alkylation catalyst is contacted with the entire alkylate-containing product stream in the presence of hydrogen.

4. A process according to claim 1 wherein the process is carried out in an apparatus comprising two or more solid acid alkylation catalyst-containing reactors, and in which process
(a) at least one of said two or more reactors operates in the alkylation mode, comprising reacting the alkylatable compound and the alkylation agent to form an alkylate-containing product stream,
(b) at least one of said two or more reactors operates in the mild regeneration mode, comprising contacting said solid acid alkylation catalyst with hydrogen in the presence of alkylate formed in the reactor during its previous cycle of operation in the alkylation mode,
(c) said reactors periodically cycle back and forth between the alkylation mode and the mild regeneration mode,
(d) alkylate is recovered from the alkylate-containing product stream.

5. A process according to any one of claims 2-4 wherein the apparatus comprises two reactors in the alkylation mode and two reactors in the mild regeneration mode.

6. A process according to any one of claims 2-4 wherein the apparatus comprises three reactors in the alkylation mode and three reactors in the mild regeneration mode.

7. A process according to any one of claims 2-6 wherein the frequency with which the reactors cycle back and forth between the alkylation mode and the mild regeneration mode ranges from once per 10 hours to 10 times per hour.

8. A process according to any one of claims 2-7 wherein the apparatus comprises one or more additional solid acid alkylation catalyst-containing reactors which can replace one or more of said two or more reactors to allow the catalyst in the replaced reactor or reactors to be subjected to high-temperature regeneration.

9. A process according to claim 8 wherein the high-temperature regeneration is performed at a temperature in the range of 423-873 K.

10. A process according to any one of claims 2-9 wherein the apparatus comprises a separation unit which separates alkylatable compound from the alkylate-containing product stream and/or the effluent from either the reactor operating in the mild regeneration mode or, if there are more reactors operating in the mild regeneration mode, from the reactor which is most downstream.

11. A process according to any one of claims 2-9 wherein at least one of said two or more reactors contains at least two catalyst beds.

12. A process according to claim 11 wherein the most downstream catalyst bed in the at least one reactor containing at least two catalyst beds is larger than the other catalyst beds in the same reactor.

13. A process according to claim 11 or 12 wherein the most downstream catalyst bed in the at least one reactor containing at least two catalyst beds contains a different catalyst than the other catalyst beds in the same reactor.

14. A process according to any one of claims 11-13 wherein alkylation agent is introduced into the at least one reactor via at least two injection points.

15. A process according to claim 14 wherein one injection point is positioned below the most upstream catalyst bed in the reactor and the other injection points are positioned below each of the subsequent catalyst beds.

16. A process according to any one of the preceding claims wherein the alkylatable compound is isobutane and the alkylation agent comprises C₃-C₅ alkenes.

17. A process according to claim 16 wherein the alkylation agent is butene or a mixture of butenes.

18. A process according to any one of the preceding claims wherein the process is conducted at a pressure in the range of 5-40 bar.

19. A process according to any one of the preceding claims wherein the alkylation reaction is performed at a temperature in the range of 293-423 K.

20. A process according to claim 19 wherein the mild regeneration is performed at a temperature, expressed in K, which does not differ from the alkylation reaction temperature by more than 20%.

21. A process according to claim 20 wherein the mild regeneration is carried out at substantially the same temperature as the alkylation reaction.

## Patentansprüche

1. Verfahren, umfassend (i) das Umsetzen einer alkylierbaren Verbindung mit einem Alkylierungsmittel über einem festen sauren Alkylierungskatalysator unter Bildung eines Alkylats und (ii) das Regenerieren des Katalysators unter milden Regenerationsbedingungen in Gegenwart von Wasserstoff und Kohlenwasserstoff, wobei der Kohlenwasserstoff wenigstens einen Teil des gebildeten Alkylats umfasst.

2. Verfahren gemäß Anspruch 1, wobei das Verfahren in einer Vorrichtung durchgeführt wird, die zwei oder mehr Reaktoren umfasst, die festen sauren Alkylierungskatalysator enthalten, und wobei in dem Verfahren
(a) wenigstens einer der zwei oder mehr Reaktoren im Alkylierungsmodus arbeitet, der das Umsetzen der alkylierbaren Verbindung und des Alkylierungsmittels unter Bildung eines alkylathaltigen Produktstroms umfasst;
(b) wenigstens einer der zwei oder mehr Reaktoren im milden Regenerationsmodus arbeitet, der das In-Kontakt-Bringen des festen sauren Alkylierungskatalysators mit wenigstens einem Teil des alkylathaltigen Produktstroms in Gegenwart von Wasserstoff umfasst;
(c) die Reaktoren periodisch zwischen dem Alkylierungsmodus und dem milden Regenerationsmodus vor und zurück wechseln;
(d) Alkylat gewonnen wird
(d1) wenn der gesamte alkylathaltige Produktstrom während der milden Regeneration mit dem festen sauren Alkylierungskatalysator in Kontakt gebracht wird; und
(d1a) wenn es nur einen Reaktor gibt, der im milden Regenerationsmodus arbeitet: aus dem Ablauf dieses Reaktors;
(d1b) wenn es zwei oder mehr Reaktoren gibt, die im milden Regenerationsmodus arbeiten: aus dem Ablauf wenigstens eines dieser Reaktoren;
(d2) wenn nur ein Teil des alkylathaltigen Produktstroms während der milden Regeneration mit dem festen sauren Alkylierungskatalysator in Kontakt gebracht wird: aus dem restlichen Teil des alkylathaltigen Produktstroms; und
(d2a) wenn es nur einen Reaktor gibt, der im mildern Regenerationsmodus arbeitet: aus dem Ablauf dieses Reaktors;
(d2b) wenn es zwei oder mehr Reaktoren gibt, die im milden Regenerationsmodus arbeiten: aus dem Ablauf wenigstens eines dieser Reaktoren.

3. Verfahren gemäß Anspruch 2, wobei der feste saure Alkylierungskatalysator im milden Regenerationsmodus in Gegenwart von Wasserstoff mit dem gesamten alkylathaltigen Produktstrom in Kontakt gebracht wird.

4. Verfahren gemäß Anspruch 1, wobei das Verfahren in einer Vorrichtung durchgeführt wird, die zwei oder mehr Reaktoren umfasst, die festen sauren Alkylierungskatalysator enthalten, und wobei in dem Verfahren
(a) wenigstens einer der zwei oder mehr Reaktoren im Alkylierungsmodus arbeitet, der das Umsetzen der alkylierbaren Verbindung und des Alkylierungsmittels unter Bildung eines alkylathaltigen Produktstroms umfasst;
(b) wenigstens einer der zwei oder mehr Reaktoren im milden Regenerationsmodus arbeitet, der das In-Kontakt-Bringen des festen sauren Alkylierungskatalysators mit Wasserstoff in Gegenwart von Alkylat, das im Reaktor während seines vorigen Zyklus beim Arbeiten im Alkylierungsmodus gebildet wurde, umfasst;
(c) die Reaktoren periodisch zwischen dem Alkylierungsmodus und dem milden Regenerationsmodus vor und zurück wechseln;
(d) Alkylat aus dem alkylathaltigen Produktstrom gewonnen wird.

5. Verfahren gemäß einem der Ansprüche 2 bis 4, wobei die Vorrichtung zwei Reaktoren im Alkylierungsmodus und zwei Reaktoren im milden Regenerationsmodus umfasst.

6. Verfahren gemäß einem der Ansprüche 2 bis 4, wobei die Vorrichtung drei Reaktoren im Alkylierungsmodus und drei Reaktoren im milden Regenerationsmodus umfasst.

7. Verfahren gemäß einem der Ansprüche 2 bis 6, wobei die Häufigkeit, mit der die Reaktoren zwischen dem Alkylierungsmodus und dem milden Regenerationsmodus vor und zurück wechseln, im Bereich von einmal pro 10 Stunden bis zehnmal pro Stunde liegt.

8. Verfahren gemäß einem der Ansprüche 2 bis 7, wobei die Vorrichtung einen oder mehrere zusätzliche Reaktoren, die festen sauren Alkylierungskatalysator enthalten, umfasst, welche einen oder mehrere der zwei oder mehr Reaktoren ersetzen können, so dass der Katalysator in dem oder den ersetzten Reaktoren einer Regeneration bei hoher Temperatur unterzogen werden kann.

9. Verfahren gemäß Anspruch 8, wobei die bei hoher Temperatur erfolgende Regeneration bei einer Temperatur im Bereich von 423 bis 873 K durchgeführt wird.

10. Verfahren gemäß einem der Ansprüche 2 bis 9, wobei die Vorrichtung eine Trenneinheit umfasst, die alkylierbare Verbindung aus dem alkylathaltigen Produktstrom und/oder den Ablauf entweder von dem Reaktor, der im milden Regenerationsmodus arbeitet, oder, wenn es mehrere Reaktoren gibt, die im milden Regenerationsmodus arbeiten, von dem Reaktor, der sich am weitesten stromabwärts befindet, abtrennt.

11. Verfahren gemäß einem der Ansprüche 2 bis 9, wobei wenigstens einer der zwei oder mehr Reaktoren wenigstens zwei Katalysatorbetten enthält.

12. Verfahren gemäß Anspruch 11, wobei das am weitesten stromabwärts befindliche Katalysatorbett in dem wenigstens einen Reaktor, der wenigstens zwei Katalysatorbetten enthält, größer ist als die anderen Katalysatorbetten in demselben Reaktor.

13. Verfahren gemäß Anspruch 11 oder 12, wobei das am weitesten stromabwärts befindliche Katalysatorbett in dem wenigstens einen Reaktor, der wenigstens zwei Katalysatorbetten enthält, einen anderen Katalysator enthält als die anderen Katalysatorbetten in demselben Reaktor.

14. Verfahren gemäß einem der Ansprüche 11 bis 13, wobei in den wenigstens einen Reaktor über wenigstens zwei Einspritzpunkte Alkylierungsmittel eingeführt wird.

15. Verfahren gemäß Anspruch 14, wobei sich ein Einspritzpunkt unter dem am weitesten stromaufwärts befindlichen Katalysatorbett in dem Reaktor befindet und sich die anderen Einspritzpunkte jeweils unter den nachfolgenden Katalysatorbetten befinden.

16. Verfahren gemäß einem der vorstehenden Ansprüche, wobei es sich bei der alkylierbaren Verbindung um Isobutan handelt und das Alkylierungsmittel C₃-C₅-Alkene umfasst.

17. Verfahren gemäß Anspruch 16, wobei es sich bei dem Alkylierungsmittel um Buten oder ein Gemisch von Butenen handelt.

18. Verfahren gemäß einem der vorstehenden Ansprüche, wobei das Verfahren unter einem Druck im Bereich von 5 bis 40 bar durchgeführt wird.

19. Verfahren gemäß einem der vorstehenden Ansprüche, wobei die Alkylierungsreaktion bei einer Temperatur im Bereich von 293 bis 423 K durchgeführt wird.

20. Verfahren gemäß Anspruch 19, wobei die milde Regeneration bei einer in K ausgedrückten Temperatur durchgeführt wird, die sich von der Temperatur der Alkylierungsreaktion um nicht mehr als 20% unterscheidet.

21. Verfahren gemäß Anspruch 20, wobei die milde Regeneration im Wesentlichen bei derselben Temperatur durchgeführt wird wie die Alkylierungsreaktion.

## Revendications

1. Procédé comprenant (i) la réaction d'un composé alkylable avec un agent d'alkylation sur un catalyseur d'alkylation acide solide pour former un alkylate, et (ii) la régénération dudit catalyseur dans des conditions de régénération douces en présence d'hydrogène et d'hydrocarbure, dans lequel l'hydrocarbure comprend au moins une partie de l'alkylate formé.

2. Procédé selon la revendication 1, dans lequel le procédé est réalisé dans un appareil comprenant deux réacteurs contenant un catalyseur d'alkylation acide solide
ou plus et dans lequel
(a) au moins un desdits deux réacteurs ou plus fonctionne en mode alkylation, comprenant la réaction du composé alkylable et de l'agent d'alkylation, afin de former un courant de produit contenant un alkylate,
(b) au moins un desdits deux réacteurs ou plus fonctionne en mode régénération douce, comprenant la mise en contact dudit catalyseur d'alkylation acide solide avec au moins une partie du courant de produit contenant un alkylate en présence d'hydrogène,
(c) lesdits réacteurs effectuent périodiquement des cycles d'aller-retour entre le mode alkylation et le mode régénération douce.
(d) l'alkylate est récupéré
(d1) si la totalité du courant de produit contenant un alkylate est mis en contact avec le catalyseur d'alkylation acide solide pendant la régénération douce, et
(d1a) s'il n'y a qu'un réacteur fonctionnant en mode régénération douce, à partir de l'effluent de ce réacteur,
(d1b) s'il y a deux réacteurs ou plus travaillant en mode régénération douce, à partir de l'effluent d'au moins un de ces réacteurs,
(d2) si seulement une partie du courant de produit contenant un alkylate est mise en contact avec ledit catalyseur d'alkylation acide solide pendant la régénération douce, à partir de la partie restante du courant de produit contenant un alkylate et
(d2a) s'il n'y a qu'un réacteur fonctionnant en mode régénération douce, à partir de l'effluent de ce réacteur,
(d2b) s'il y a deux réacteurs ou plus fonctionnant en mode régénération douce, à partir de l'effluent d'au moins un de ces réacteurs.

3. Procédé selon la revendication 2, dans lequel en mode régénération douce, le catalyseur d'alkylation acide solide est mis en contact avec la totalité du courant de produit contenant un alkylate en présence d'hydrogène.

4. Procédé selon la revendication 1, dans lequel le procédé est réalisé dans un appareil comprenant deux réacteurs contenant un catalyseur d'alkylation acide solide
ou plus, et dans lequel
(a) au moins un desdits deux réacteurs ou plus fonctionne en mode alkylation, comprenant la réaction du composé alkylable et de l'agent d'alkylation pour former un courant de produit contenant un alkylate,
(b) au moins un desdits deux réacteurs ou plus fonctionne en mode régénération douce, comprenant la mise en contact dudit catalyseur d'alkylation acide solide avec de l'hydrogène, en présence d'un alkylate formé dans le réacteur pendant son cycle précédent de fonctionnement en mode alkylation,
(c) lesdits réacteurs effectuent périodiquement des cycles d'aller-retour entre le mode alkylation et le mode régénération douce.
(d) l'alkylate est récupéré du courant de produit contenant un alkylate.

5. Procédé selon l'une quelconque des revendications 2 à 4, dans lequel l'appareil comprend deux réacteurs en mode alkylation et deux réacteurs en mode régénération douce.

6. Procédé selon l'une quelconque des revendications 2 à 4, dans lequel l'appareil comprend trois réacteurs en mode alkylation et trois réacteurs en mode régénération douce.

7. Procédé selon l'une quelconque des revendications 2 à 6, dans lequel la fréquence à laquelle les réacteurs effectuent des cycles d'aller-retour entre le mode alkylation et le mode régénération douce est comprise entre une fois toutes les 10 heures et 10 fois par heure.

8. Procédé selon l'une quelconque des revendications 2 à 7, dans lequel l'appareil comprend un ou plusieurs réacteurs contenant un catalyseur d' alkylation acide solide supplémentaire, qui peut remplacer un ou plusieurs desdits deux réacteurs ou plus, afin de permettre au catalyseur dans le ou les réacteurs remplacés d'être soumis à une régénération à haute température.

9. Procédé selon la revendication 8, dans lequel la régénération à haute température est effectuée à une température comprise dans la plage allant de 423 à 873 K.

10. Procédé selon l'une quelconque des revendications 2 à 9 dans lequel l'appareil comprend une unité de séparation qui sépare le composé alkylable du courant de produit contenant un alkylate et/ou de l'effluent du réacteur fonctionnant en mode régénération douce, ou s'il y a plusieurs réacteurs fonctionnant en mode régénération douce, du réacteur qui est le plus en aval.

11. Procédé selon l'une quelconque des revendications 2 à 9 dans lequel au moins un desdits deux réacteurs ou plus contient au moins deux lits de catalyseur.

12. Procédé selon la revendication 11 dans lequel le lit de catalyseur le plus en aval dans ledit au moins un réacteur contenant au moins deux lits de catalyseur est plus grand que les autres lits de catalyseur dans le même réacteur.

13. Procédé selon la revendication 11 ou 12, dans lequel le lit de catalyseur le plus en aval dans ledit au moins un réacteur contenant au moins deux lits de catalyseurs contient un catalyseur différent des autres lits de catalyseurs dans le même réacteur.

14. Procédé selon l'une quelconque des revendications 11 à 13, dans lequel l'agent d'alkylation est introduit dans ledit au moins un réacteur par le biais d'au moins deux points d'injection.

15. Procédé selon la revendication 14, dans lequel un point d'injection est placé sous le lit de catalyseur le plus en amont dans le réacteur et les autres points d'injection sont placés sous chacun des lits de catalyseur successifs.

16. Procédé selon l'une quelconque des revendications précédentes dans lequel le composé alkylable est un isobutane et l'agent d'alkylation comprend des alcènes en C₃-C₅.

17. Procédé selon la revendication 16, dans lequel l'agent d'alkylation est un butène
ou un mélange de butènes.

18. Procédé selon l'une quelconque des revendications précédentes, dans lequel le procédé est conduit à une pression comprise dans la plage allant de 5 à 40 bars.

19. Procédé selon l'une quelconque des revendications précédentes, dans lequel la réaction d'alkylation est réalisée à une température comprise dans la plage allant de 293 à 423 K.

20. Procédé selon la revendication 19, dans lequel la régénération douce est réalisée à une température, exprimée en K, qui ne diffère pas de la température de réaction d'alkylation de plus de 20 %.

21. Procédé selon la revendication 20, dans lequel la régénération douce est réalisée sensiblement à la même température que la réaction d'alkylation.
